# EUROPÄISCHE PATENTSCHRIFT

(11) **EP 1 268 370 B1**
(45) Veröffentlichungstag und Bekanntmachung des Hinweises auf die Patenterteilung: **16.06.2004**
(21) Anmeldenummer: 01927780.5
(22) Anmeldetag: 22.03.2001
(51) Int. Cl.: C07C 2/08

(54) **VERFAHREN ZUR HERSTELLUNG VON OLIGOMEREN**
METHOD FOR PRODUCING OLIGOMERS
PROCEDE DE PREPARATION D'OLIGOMERES

(30) Priorität: 25.03.2000 DE 10015002
(43) Veröffentlichungstag der Anmeldung: 02.01.2003
(73) Patentinhaber: BASF AKTIENGESELLSCHAFT, 67056 Ludwigshafen (DE)
(72) Erfinder: SCHULTZ, Ralf, 67346 Speyer (DE); HEIDEMANN, Thomas, 68519 Viernheim (DE); SCHWAB, Peter, 67098 Bad Dürkheim (DE); ZEHNER, Peter, 67071 Ludwigshafen (DE)
(74) Vertreter: Pohl, Michael, Dr.
(86) Internationale Anmeldenummer: PCT/EP2001/003262
(87) Internationale Veröffentlichungsnummer: WO 2001/072670

(56) Entgegenhaltungen:
- WO-A-99/25668
- DE-A- 19 922 038

## Beschreibung

Die vorliegende Erfindung betrifft ein Verfahren zur Herstellung von im Wesentlichen unverzweigten Oligomeren von Alkenen mit 4 bis 6 Kohlenstoffatomen ausgehend von den Alkenen oder solche Alkene enthaltenden Kohlenwasserstoffströmen an einem Nickel-haltigen heterogenen Katalysator in einem adiabatisch betriebenen Reaktor bei Temperaturen von 20 bis 300°C und Drücken von 1 bis 100 bar.

Alkene mit 2 bis 6 Kohlenstoffatomen und deren Gemische, insbesondere Alkene mit 4 Kohlenstoffatomen, stehen in großen Mengen sowohl aus FCC-Anlagen als auch aus Steamcrackern zur Verfügung. Der jeweilige C₄-Schnitt, d.h. das Gemisch aus Butenen und Butanen eignet sich nach Abtrennung des iso-Butens sehr gut zur Herstellung von Oligomeren, insbesondere Octenen und Dodecenen. Sowohl die Octene als auch Dodecene können durch Hydroformylierung und nachfolgende Hydrierung zu den entsprechenden Alkoholen z.B. für die Herstellung von Weichmachern Verwendung finden.

Dabei wirkt sich der Verzweigungsgrad des weichmacheralkohols maßgeblich auf die Eigenschaften des Weichmachers aus. Der Verzweigungsgrad wird durch den ISO-Index beschrieben, welcher die mittlere Zahl der Methylverzweigungen in einer Fraktion von isomeren Verbindungen mit Kohlenwasserstoffketten, hier speziell von Alkenen und von den von diesen abgeleiteten Alkoholen, angibt. So tragen z.B. n-Octene mit 0, Methylheptene mit 1 und Dimethylhexene mit 2 zum ISO-Index einer Fraktion bei. Mit anderen Worten: Je niedriger der ISO-Index ist, um so linearer sind die Moleküle in der jeweiligen Fraktion aufgebaut. Je höher wiederum die Linearität ist, d.h. je niedriger der ISO-Index, um so höher sind die Ausbeuten in der Oxierung und umso besser sind die Eigenschaften des damit hergestellten Weichmachers.

Die ältere deutsche Patentanmeldung mit dem Aktenzeichen 199 10 103.5 lehrt ein Verfahren zur Oligomerisierung von C₆-Alkenen durch Umsetzung eines C₆-Alkene enthaltenden Reaktionsgemisches an einem Nickel enthaltenden Festbettkatalysator, wobei es darauf ankommt, dass nur maximal 30 Gew.-% der C₆-Alkene, bezogen auf das Reaktionsgemisch, umgesetzt werden. Unumgesetztes C₆-Alken kann von oligomeren Produkten abgetrennt und in die Umsetzung zurückgeführt werden.

Aus der WO-A 99/25668 ist ferner ein Verfahren zur Herstellung von im Wesentlichen unverzweigten Octenen und Dodecenen bekannt, bei dem man 1-Buten und/oder 2-Buten und Butan enthaltende Kohlenwasserstoffströme an einem heterogenen Katalysator, der Nickel enthält, umsetzt. Dabei werden solche Mengen des von dem Reaktionsgemisch abgetrennten Butans und nicht umgesetzten Butens in die Oligomerisierungsreaktion zurückführt, dass der maximale Gehalt an Oligomeren im umgesetzten Reaktionsgemisch 25 Gew.-% an keiner Stelle im Reaktor übersteigt.

Die Oligomerisierung von niedermolekularen Alkenen wird in aller Regel von einer merklichen Wärmetönung begleitet. Bei einer Reaktion an einem heterogenen Katalysator wird weiterhin unter adiabatischen Bedingungen praktisch die gesamte Reaktionswärme durch das Reaktionsgemisch aus dem Reaktor abgeführt. Das bedeutet, dass sich der Durchsatz durch den Reaktor und damit auch der Umsatz in der Zeiteinheit wesentlich danach richten müssen, wieviel Wärme aus dem Reaktor abzuführen sein wird. Daher dosiert der Fachmann den Alkenstrom gerade so in den Reaktor und stellt dazu die übrigen Reaktionsparameter gerade derart ein, dass nach einmaligem Durchleiten des Alkenstroms durch den Reaktor ("im geraden Durchgang") im austretenden Produktstrom die gewünschte Oligomerenkonzentration vorliegt. Diesen Optimierungsschritt nimmt er normalerweise in jedem Einzelfall von Neuem, und er optimiert die genannten Parameter in einer Vielzahl von Vorversuchen. Unter diesen Umständen wird jedoch nur ein Nutzungsgrad erreicht, der noch nicht befriedigen kann.

Es war daher die Aufgabe der vorliegenden Erfindung, ein heterogenkatalytisches adiabatisches Verfahren für die Herstellung von Alken-Oligomeren bereitzustellen, bei dem - bei gleichzeitigem hohem Umsatz und hoher Produktselektivität - die Temperatur im Reaktor flexibler gesteuert werden kann als in den bekannten derartigen Verfahren.

Diese Aufgabe wurde erfindungsgemäß gelöst durch ein Verfahren zur Herstellung von im Wesentlichen unverzweigten Oligomeren von Alkenen mit 4 bis 6 Kohlenstoffatomen ausgehend von den Alkenen oder einem solche Alkene enthaltenden Kohlenwasserstoffstrom an einem Nickel-haltigen heterogenen Katalysator in einem adiabatisch betriebenen Reaktor bei Temperaturen von 20 bis 300°C und Drücken von 1 bis 100 bar, indem man einen ersten Teilstrom des Austrags aus dem Reaktor auf die Oligomeren aufarbeitet und den zweiten Teilstrom zusammen mit frischem Alken oder einem frischen, solche Alkene enthaltenden Kohlenwasserstoffstrom, in den Reaktor zurückführt.

Unter "Oligomeren" werden in dieser Anmeldung Dimere, Trimere und höhere Produkte der Zusammenlagerung von Alkenen mit 4 bis 6 Kohlenstoffatomen verstanden, die bis zu 18, vorzugsweise 12 und besonders bevorzugt 8 Kohlenstoffatome aufweisen.

Die vorliegende Erfindung basiert auf der Erkenntnis, dass es für die Aktivität der verwendeten Nickelkatalysatoren, für den Gesamtumsatz und insbesondere die Selektivität des Verfahrens in weiten Grenzen ohne Bedeutung ist, wenn im Verfahren gebildete oligomere Produkte mit oligomerenfreiem Ausgangs-Alken vermischt werden und das Gemisch dann den Reaktor passiert.

Als Katalysatoren kommen allgemein Nickel enthaltende Katalysatoren in Betracht, die bekanntermaßen eine geringe Oligomeren-Verzweigung bewirken: Vgl. z.B. Catalysis Today, 6, 329 (1990), insbesondere die Seiten 336-338, sowie die in der WO-A 95/14647 und der älteren deutschen Patentanmeldung mit dem Aktenzeichen 199 57 173.2 zum Stand der Technik zitierten Literaturstellen, wobei insbesondere auf diese Literaturstellen hiermit ausdrücklich Bezug genommen wird.

Beispielhaft sind als solche Nickel enthaltenden Oligomerisierungskatalysatoren - in denen das Nickel in der Regel in oxidischer Form vorliegt - zu nennen:
* Nickel auf Siliciumdioxid,
* Nickel auf Siliciumdioxid-Aluminiumoxid,
* Nickel auf Siliciumdioxid-Aluminiumoxid-Schichtsilikaten, wie Glimmer und Tonerden, insbesondere Montmorillonite,
* Nickel auf Zeolith-Trägern, wie Mordenit, Faujasit, Zeolith X, Zeolith Y, Zeolith ZSM-5 oder andere Zeolithe vom ZSM-Typ, Zeolithe mit MCM-41 Struktur oder CZS-1 Struktur,
* Nickel auf Aluminiumoxid, gegebenenfalls zusammen mit Anionen vor allem anorganischer Säure wie Schwefel-, Phosphor-, Borsäure,
* Nickel auf Zirkoniumoxid, das mit Säuren, wie Schwefelsäure, Phosphorsäure oder Borsäure behandelt ist,
* NiO/ZrO₂/SO₄/SiO₂-Systeme,
* Nickel auf sulfatiertem Titandioxid.

Die erfindungsgemäß verwendeten Nickel enthaltenden Oligomerisierungskatalysatoren werden im Folgenden kurz als "Ni-Katalysatoren" bezeichnet.

Nach einer bevorzugten Ausführungsform des erfindungsgemäßen Verfahrens wird die Oligomerisierung in flüssiger Phase durchgeführt unter Verwendung der in der WO-A 95/14647 und der älteren deutschen Patentanmeldung mit dem Aktenzeichen 199 57 173.2 beschriebenen und beanspruchten Ni-Katalysatoren. Deshalb wird auf diese Schriften ausdrücklich Bezug genommen und deren Angaben hinsichtlich des Verfahrens und der Ni-Katalysatoren sollen als hier inkorporiert gelten.

Die in der WO-A 95/14647 beschriebenen Ni-Katalysatoren bestehen in ihren katalytisch aktiven Masse im Wesentlichen, d.h. ohne Berücksichtigung von Verunreinigungen, die durch Ausgangs- oder Prozeßchemikalien bei der Ni-Katalysatorherstellung eingeschleppt werden, aus Nickeloxid, Siliciumdioxid, Titandioxid und/oder Zirkondioxid sowie gegebenenfalls Aluminiumoxid. Diese Ni-Katalysatoren enthalten 10 bis 70 Gew.-% Nickeloxid, 5 bis 30 Gew.-% Titandioxid und/oder Zirkondioxid, 0 bis 20 Gew.-% Aluminiumoxid und als Rest bis zu 100 Gew.-% Siliciumdioxid. Man erhält sie durch Fällung der Ni-Katalysatormasse bei pH 5 bis 9, indem man eine Nickelnitrat enthaltenden wäßrigen Lösung zu einer Alkaliwasserglaslösung gibt, die noch Titandioxid und/oder Zirkondioxid enthält. Anschließend wird die so erhaltene Ni-Katalysatormasse filtriert, getrocknet und bei 350 bis 650°C getempert.

Bei den Ni-Katalysatoren der älteren deutschen Patentanmeldung mit dem Aktenzeichen 199 57 173.2 handelt es sich im Wesentlichen um Aluminiumoxid, das mit einer Nickelverbindung und einer Schwefelverbindung beaufschlagt wurde, wobei im fertigen Ni-Katalysator ein molares Verhältnis von Schwefel zu Nickel von 0,25 : 1 bis 0,38 : 1 vorliegt.

Die erfindungsgemäß verwendeten Ni-Katalysatoren werden vorzugsweise in einem Festbett angeordnet und haben deshalb vorzugsweise stückige Form: z.B. Tabletten (5 mm x 5 mm, 5 mm x 3 mm, 3 mm x 3 mm), Ringe (7 mm x 7 mm x 3 mm, 5 mm x 5 mm x 2 mm, 5 mm x 2 mm x 2 mm) oder Stränge bzw. Sternenstränge (1,5 mm-Durchmesser, 3 mm-Durchmesser, 5 mm-Durchmesser). Die vorstehenden Größenangaben und Formkörpertypen sind lediglich beispielhaft und stellen keine Einschränkung des Gegenstandes der vorliegenden Erfindung dar.

Im einzelnen führt man das erfindungsgemäße Verfahren so aus, dass man die Alkene mit 4 bis 6 Kohlenstoffatomen (im Folgenden kurz als "Alkene" genannt) oder deren Gemische mit Alkanen, bevorzugt in flüssiger Phase, über den genannten Ni-Katalysatoren umsetzt.

Vorzugsweise verwendet man als Alken die einfach ungesättigten Butene, Pentene und Hexene, insbesondere jeweils für sich allein 1-Buten, 2-Buten, 1-Penten, 2-Penten, 1-Hexen oder 3-Hexen.

Bevorzugt setzt man als Alkene enthaltende Kohlenwasserstoffströme solche mit mindestens einem Alken aus der Gruppe: n-Buten, n-Penten, n-Hexen ein.

Das erfindungsgemäße Verfahren erlaubt die Verwendung von Einsatzstoffströme, deren Gehalt an Alken normalerweise bei 5 bis 100, vorzugsweise bei 30 bis 100 und besonders bevorzugt bei 50 bis 100 Gew.-% liegt.

Ganz besonders geeignet ist das erfindungsgemäße Verfahren für die Umsetzung von Alkenen, die im Gemisch mit Alkanen vorliegen, wobei Alkene und Alkane insbesondere jeweils 4 Kohlenstoffatome aufweisen. Geeignete derartige C₄-Kohlenwasserströme sind z.B. Gemische mit folgender Zusammensetzung:

| | |
|---|---|
| Butan | 10 bis 90 Gew.-% |
| Buten | 10 bis 90 Gew.-%, |

wobei die Buten-Fraktion folgende Zusammensetzung haben kann:

| | |
|---|---|
| 1-Buten | 1 bis 50 Gew.-% |
| cis-2-Buten | 1 bis 50 Gew.-% |
| trans-2-Buten | 1 bis 99 Gew.-% |
| iso-Buten | 1 bis 5 Gew.-% |

Als besonders bevorzugter Einsatzstoff wird das sogenannte Raffinat II verwendet. Es handelt sich dabei um ein Buten-haltiges C₄-Kohlenwasserstoffgemisch, wie es aus dem C₄-Schnitt von Crackern erhalten wird, nachdem höher ungesättigte Kohlenwasserstoffe, wie Dialkene, insbesondere 1,3-Butadien, oder Acetylen und anschließend das darin enthaltene iso-Buten abgetrennt wurden. Eine typische Zusammensetzung für ein Raffinat II ist z.B.:

| | |
|---|---|
| iso-, n-Butan | 26 Gew.-% |
| iso-Buten | 1 Gew.-% |
| 1-Buten | 26 Gew.-% |
| trans-2-Buten | 31 Gew.-% |
| cis-2-Buten | 16 Gew.-% |

Die C₄-Kohlenwasserstoffströme können weiterhin gemäß der DE-A 39 14 817 durch Hydrierung bzw. Absorption an Molekularsieb von Butadien, schwefelhaltigen und sauerstoffhaltigen Verbindungen, wie Alkoholen, Aldehyden, Ketonen oder Ethern befreit werden.

Die Oligomerisierungsreaktion findet in der Regel bei Temperaturen von 30 bis 280°C, vorzugsweise von 30 bis 140°C und insbesondere von 40 bis 130°C und einem Druck von in der Regel 1 bis 300 bar, vorzugsweise von 5 bis 100 bar und insbesondere von 20 bis 70 bar statt. Der Druck wird dabei zweckmäßigerweise so ausgewählt, dass das Einsatzkohlenwasserstoffgemisch bei der eingestellten Temperatur überkritisch und insbesondere flüssig vorliegt.

Der Reaktor ist in der Regel ein mit dem Ni-Katalysator beschickter zylindrischer Reaktor; alternativ kann eine Kaskade aus mehreren, vorzugsweise zwei, hintereinander geschalteten derartigen Reaktoren eingesetzt werden.

In dem Reaktor oder den einzelnen Reaktoren der Reaktorkaskade kann der Ni-Katalysator in einem einzigen oder in mehreren Ni-Katalysator-Festbetten angeordnet sein. Außerdem ist es möglich, in den einzelnen Reaktoren der Kaskade unterschiedliche Ni-Katalysatoren einzusetzen, obgleich die Anwendung des gleichen Ni-Katalysators in sämtlichen Reaktoren der Kaskade bevorzugt wird.

Weiterhin können in den einzelnen Reaktoren der Reaktorkaskade unterschiedliche Reaktionsbedingungen hinsichtlich Druck und/oder Temperatur im Rahmen der obengenannten Druck- und Temperaturbereiche eingestellt werden.

Im Reaktor oder der Reaktorkaskade wird das Festbett des Ni-Katalysator von dem flüssigen Reaktionsgemisch z.B. von oben nach unten durchströmt.

Der beschriebene erfindungsgemäß verwendete einzige Reaktor und die beschriebene Reaktorkaskade für die Umsetzungen am Festbett des Ni-Katalysators werden im Folgenden unter dem Begriff "Festbett-Reaktor" subsummiert.

Unter einer adiabatischen Reaktionsführung oder Betriebsweise wird im technischen Sinne eine Reaktionsführung oder Betriebsweise verstanden, bei der, abgesehen von dem durch natürliche Wärmeleitung und Wärmeabstrahlung vom Reaktor an die Umgebung abgegebenen Teil der Reaktionswärme, die gesamte Reaktionswärme vom Reaktionsgemisch aufgenommen und mit diesem aus dem Reaktor abgeführt wird.

Im Gegensatz dazu wird bei der isothermen Reaktionsführung oder Betriebsweise im technischen Sinne die Abfuhr der Reaktionswärme aus dem Reaktor mittels Kühl- oder Thermostatisiervorrichtungen über das durch natürliche Wärmeleitung oder Wärmeabstrahlung gegebene Maß hinaus gezielt forciert. Dabei kann ein - wenn auch vernachlässigbar kleiner - Teil der Reaktionswärme praktisch unvermeidlich mit dem erwärmten Reaktionsgemisch ausgetragen werden.

Alternativ zu den genannten Festbett-Reaktoren kann das erfindungsgemäße Verfahren in anderen Reaktoren durchgeführt werden, die der Fachmann für derartige heterogenkatalysierte adiabatische Umsetzungen kennt, beispielsweise Rührkessel oder Schlaufenreaktoren (vgl. M. Baerns, H. Hoffmann, A. Renken, Chemische Reaktionstechnik, Thieme Verlag Stuttgart 1987, Seite 237 ff.).

Der Umsatz zu den gewünschten Oligomeren liegt beim erfindungsgemäßen Verfahren in der Regel bei 10 bis 100, und vorzugsweise bei 50 bis 100 %, bezogen auf die eingesetzten Alkene.

Bevorzugt beträgt der Anteil an Oligomeren am Austrag aus dem Reaktor 1 bis 80 Gew.-%, besonders bevorzugt 10 bis 50 Gew.-% und insbesondere 15 bis 25 Gew.-%.

Nach dem Verlassen der ein- oder mehrstufigen Reaktionszone wird der rohe Produktstrom in einen ersten und einen zweiten Produkt-Teilstrom aufgeteilt.

Bevorzugt beträgt der erste Teilstrom 1 bis 50 Gew.-% des Austrags aus dem Reaktor, besonders bevorzugt 1 bis 20 Gew.-% des Austrags aus dem Reaktor und insbesondere 1 bis 10 Gew.-% des Austrags.

Der erste Produkt-Teilstrom wird in an sich bekannter Weise, vorzugsweise destillativ, auf die gebildeten Oligomeren aufgearbeitet. Dabei werden Restmengen der nicht umgesetzten Alkene und die gegebenenfalls begleitenden Alkane als "Purge-Strom" abgetrennt.

Der Purge-Strom hat Restgehalte an Alken von in der Regel 5 bis 20 Gew.-% und hat nur noch geringen Wert als Grundstoff für organische Synthesen. Er wird daher in der Regel sonstigen Verwendungszwecken zugeführt, etwa einem Crackprozess, der erneut zu im erfindungsgemäßen Verfahren verwertbaren Alkenen führen kann.

Der Purgestrom kann auch teilweise oder ganz in den ersten Reaktor zurückgeführt werden. Bei seinem niedrigen Gehalt an reaktivem Alken besteht seine Wirkung beispielsweise darin, den Stoffstrom durch den Reaktor zu vergrößern, also das Alken zu verdünnen, und damit letztlich zur Temperatursteuerung im Reaktor beizutragen. Weiterhin lässt sich so die Obergrenze für den Oligomerengehalt im Produktstrom leichter kontrollieren.

Der zweite Produkt-Teilstrom wird bei praktisch unveränderter Zusammensetzung in das Verfahren zurückgeführt. Die Temperatur des zweiten Produkt-Teilstroms kann vor der Zufuhr in den Reaktor mit hierzu bekannten Vorrichtungen wie Wärmetauschern auf die gewünschte Temperatur gestellt werden.

Da Verfahren wie das erfindungsgemäße üblicherweise auf Einsatz-stoffe einer bestimmten Beschaffenheit und Zusammensetzung optimiert sind, ist es in der Regel, auch für einen hinreichenden Umsatz, erforderlich, den Anteil des Alkens im zweiten Produkt-Teilstrom wieder in die Nähe oder auf den Ausgangswert am Reaktoreingang zu erhöhen. Dazu wird dem zweiten Teilstrom erfindungsgemäß frisches Alken, das mit einem oder mehreren Alkanen vermischt sein kann, zudosiert.

Bevorzugt beträgt das Gewichtsverhältnis von zweitem Teilstrom zu frischem Alken oder dem frischen, ein solches Alken enthaltenden Kohlenwasserstoffstrom, 1 : 1 bis 50 : 1, besonders bevorzugt 2 : 1 bis 20 : 1 beträgt und insbesondere 5 : 1 bis 20 : 1.

In welchem Verhältnis der Strom des frischen Alkens und der gegebenenfalls zurückgeführte Anteil des Purge-Stroms zum zweiten Teilstrom vor der Zufuhr in den Reaktor stehen müssen, kann der Fachmann im Hinblick auf die gewünschte Oligomerenausbeute und -selektivität und die dazu einzustellende Temperatur im Reaktorinneren leicht anhand einfacher Vorversuche ermitteln.

Weiterhin kann der Strom des bei der Aufarbeitung des ersten Teilstroms zurückgewonnenen, unumgesetzten Alkens mit 4 bis 6 Kohlenstoffatomen zusammen mit den gegebenenfalls begleitenden Alkanen in den Reaktor zurückführt werden.

Die Zufuhr des zweiten Produkt-Teilstroms und des frischen Stroms des Alkens in den Reaktor kann so erfolgen, dass die Ströme gleichzeitig einzeln, etwa über getrennte Leitungen, oder nach vorherigem Vermischen in den Reaktor geleitet werden.

Vor der Zufuhr in den Reaktor kann die Temperatur jedes einzelnen Stromes oder das Gemisch der Ströme der Einsatzstoffe mit zu diesem Zweck an sich bekannten Vorrichtungen wie Wärmetauschern eingestellt werden.

Ist der Ni-Katalysator im Reaktor in mehreren Festbetten angeordnet, können die vermischten Einsatzstoffströme aufgeteilt und an mehreren Stellen, z.B. vor einem ersten Festbett in Fließrichtung des Reaktionsgemisches und/oder zwischen einzelnen Ni-Katalysatorfestbetten, in den Reaktor eingeleitet werden. Bei Verwendung einer Reaktorkaskade beispielsweise ist es möglich, die vermischten Einsatzstoffströme sowohl vollständig dem ersten Reaktor der Kaskade zuzuführen oder über mehrere Zuleitungen auf die einzelnen Reaktoren der Kaskade, wie für den Fall des Einzelreaktors beschrieben, verteilt werden.

Mit dem erfindungsgemäßen Verfahren werden Gehalte an Oligomeren von 5 bis 100, vorzugsweise 10 bis 60 und vor allem 15 bis 30 Gew.-%, bezogen auf den gesamten Produktstrom, erhalten. Zur Erzielung eines solchen Oligomerengehaltes wird in der Regel ein Gewichtsverhältnis von Rückführstrom zu frisch zugeführtem Einsatzkohlenwasserstoffstrom von 0,5 bis 10, vorzugsweise von 1 bis 7, insbesondere von 1 bis 4 eingestellt, wobei sich diese Angaben auf den stationären Zustand des Reaktionssystems beziehen.

Nach unten besteht an sich keine Grenze für den Oligomerengehalt im Reaktionsgemisch, doch wird das Verfahren bei der Wahl eines sehr geringen Oligomerengehaltes wegen des übermäßig groß werdenden Rückführstromes unwirtschaftlich. Deshalb wird in der Regel eine untere Grenze von 10 Gew.-% Oligomeren, im umgesetzten Reaktionsgemisch vor dessen Aufteilung nicht unterschritten.

Durch das erfindungsgemäße Verfahren ist es dem Fachmann möglich, flexibel auf die Wärmeentwicklung im Reaktor zu reagieren, weil er das Verhältnis der Zufuhrströme zum Reaktor in weiten Grenzen frei wählen kann. Außerdem kann er vor allem die Temperatur des zweiten Teilstroms gezielt an seinen Bedarf anpassen.

Mit der erfindungsgemäßen Verfahren hat er weiterhin die Möglichkeit, die Erwärmung des Stoffstroms durch den Reaktor ("Temperaturhub") bis in die Nähe einer quasi-isothermen Reaktionsführung abzusenken, wodurch die Temperatur im Reaktor noch besser beherrschbar wird.

In den folgenden Beispielen ist das erfindungsgemäße Verfahren im einzelnen demonstriert.

### Beispiele

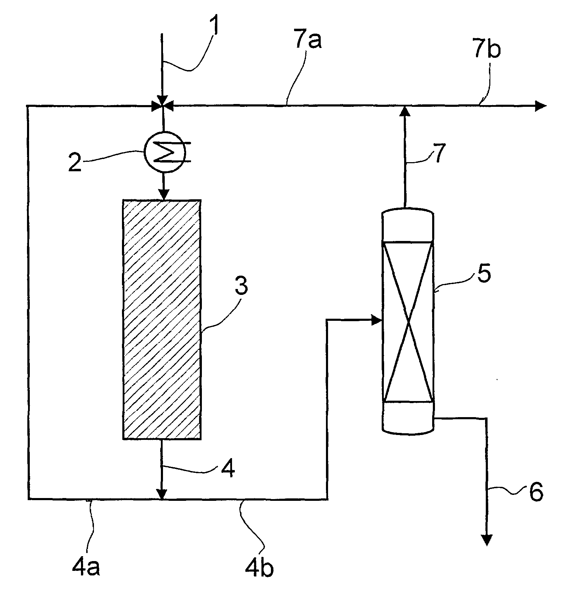

Abbildung 1 zeigt das Schema einer Vorrichtung, in der das erfindungsgemäße Verfahren ausgeführt werden kann. Annahme: Die Umsetzung befinde sich bereits im stationären Zustand. Frisches Alken wird über (1) und Wärmetauscher (2) dem Reaktor (3) zugeführt. Der rohe Produktstrom in Leitung (4) wird teilweise über Leitung (4a) ohne Aufarbeitung in den Reaktor zurückgeführt. Der andere Teil des rohen Produktstroms wird über (4b) zur Kolonne (5) geleitet, wo er in die oligomeren Produkte (6) und ein Kopfprodukt (7) aufgetrennt wird. Das Kopfprodukt (7) wird ganz oder teilweise über (7b) aus dem Verfahren ausgeschleußt, wobei der gegebenenfalls verbleibende Teil über (7a) zusammen mit dem frischen Alken (1) und dem Teilstrom aus (4a) über den Wärmetauscher in den Reaktor zurückgeführt wird.

### A) Ni-Katalysatoren

### Ni-Katalysator I

Ein Material der Zusammensetzung 50 Gew.-% NiO, 12,5 Gew.-% TiO₂, 33,5 Gew.-% SiO₂ und 4 Gew.-% Al₂O₃ gemäß der WO-A 95/14647 wurden, wie dort beschrieben, zu 3 mm-Sternensträngen verformt.

### Ni-Katalysator II

Ein Material der Zusammensetzung 8,9 Gew.-% Ni, 1,6 Gew.-% S und dem Rest zu 100 Gew.-% Al₂O₃ gemäß der älteren deutschen Patentanmeldung mit dem Aktenzeichen 199 57 173.2 wurden, wie dort beschrieben, in Form von 3 mm-Sternensträngen hergestellt.

### Ni-Katalysator III

Ein Material der Zusammensetzung 50 Gew.-% NiO, 12,5 Gew.-% TiO₂, 33,5 Gew.-% SiO₂, 4 Gew.-% Al₂O₃ gemäß der WO-A 95/14647 wurde, wie dort beschrieben, zu einem unverformten Sprühgranulat (Partikelgröße 100-350 µm) verarbeitet.

### B) Verwendete Kohlenwasserstoffe mit 4 bis 6 Kohlenstoffatomen

Das verwendete Raffinat II hatte folgende Zusammensetzung:

| | |
|---|---|
| iso-Butan | 3 Gew.-% |
| n-Butan | 15 Gew.-% |
| iso-Buten | 2 Gew.-% |
| 1-Buten | 30 Gew.-% |
| trans-2-Buten | 32 Gew.-% |
| cis-2-Buten | 18 Gew.-% |

Das verwendete "C₆-Gemisch" hatte folgende Zusammensetzung:

| | |
|---|---|
| 1-Hexen | <0,1 Gew.-% |
| trans-2-Hexen | <0,1 Gew.-% |
| cis-2-Hexen | <0,1 Gew.-% |
| trans-3-Hexen | 86,2 Gew.-% |
| cis-3-Hexen | 12,2 Gew.-% |
| 2-Methylpent-2-en | 1,1 Gew.-% |
| Pentene | 0,5 Gew.-% |

### C) Oligomerisierungen

### Beispiel 1

Raffinat II als Feed wurde an einem Festbett von 83 g Ni-Katalysator I der Füllhöhe 22 cm in einem Rohrreaktor (Innendurchmesser 28 mm, Länge 50 cm) im geraden Durchgang bzw. unter Rückführung umgesetzt. Weitere Versuchparameter und die Ergebnisse der Versuche sind in Tabelle 1 zusammengestellt.

**Tabelle 1**

| | T [°C] | Feed [g/h] | Rückführung [g/h] | Ausbeute [Gew.-%] | Selektivität C8 [%] |
|---|---|---|---|---|---|
| ohne Rückführung | 80 | 40 | 0 | 28,1 | 68,7 |
| | 80 | 66 | 0 | 24 | 72,1 |
| | 80 | 115 | 0 | 17,1 | 77,9 |
| mit Rückführung | 80 | 40 | 820 | 27,6 | 70 |
| | 80 | 62 | 870 | 23 | 73,7 |
| | 80 | 100 | 840 | 17,9 | 77,3 |

Erläuterung der Bezeichnungen in der Tabelle:
- T: Temperatur der Umsetzung
- Rückführung: Erfindungsgemäßes Rückführen eines Oligomeren-haltigen Teilstroms
- Ausbeute: Ausbeute an Oligomeren bezogen auf den gesamten Rohproduktaustrag
- Selektivität C8: Anteil von (hier:) C₈-Isomeren an den Oligomeren im gesamten Produktstrom

### Beispiel 2

Raffinat II als Feed wurde an einem Festbett von 95 g Ni-Katalysator II der Füllhöhe 23 cm in einem Rohrreaktor (Innendurchmesser 28 mm, Länge 50 cm) im geraden Durchgang bzw. unter Rückführung umgesetzt. Weitere Versuchparameter und die Ergebnisse der Versuche sind in Tabelle 2 zusammengestellt.

**Tabelle 2**

| | T [°C] | Feed [g/h] | Rückführung [g/hl | Ausbeute [Gew.-%] | Selektivität C8 [%] |
|---|---|---|---|---|---|
| ohne Rückführung | 75 | 100 | 0 | 31,1 | 83,9 |
| | 85 | 80 | 0 | 34 | 83 |
| mit Rückführung | 75 | 100 | 720 | 29 | 84,5 |
| | 85 | 80 | 710 | 32,5 | 83,5 |

Erläuterung der Bezeichnungen in der Tabelle: Vgl. Beispiel 1.

### Beispiel 3

20 g Raffinat II als Feed wurden 16 Stunden bei 80°C mit 2 g Ni-Katalysator III in einem Rührkessel umgesetzt. Die Ausbeute an Oligomeren bezogen auf den gesamten Rohproduktaustrag betrug 19 Gew.-%; der Anteil von C₈-Isomeren an diesen Oligomeren lag bei 75,1 %.

67 g Raffinat II als Feed wurde pro Stunde bei 37°C an 30 g Ni-Katalysator III in einem Fließbett umgesetzt, wobei der Ni-Katalysator von unten angeströmt und dadurch bis zu einer Höhe von 29 cm suspendiert wurde. Die Rückführung betrug 960 g/h. Die Ausbeute an Oligomeren bezogen auf den gesamten Rohproduktaustrag betrug 23,4 Gew.-%; der Anteil von C₈-Isomeren an diesen Oligomeren lag bei 73,9 %.

### Beispiel 4

C₆-Gemisch als Feed wurde an einem Festbett von 880 g Ni-Katalysator I der Füllhöhe 75 cm in einem Rohrreaktor (Innendurchmesser 48 mm, Länge 90 cm) im geraden Durchgang bzw. unter Rückführung umgesetzt. Weitere Versuchparameter und die Ergebnisse der Versuche - vor allem die Selektivität hinsichtlich der C₁₂-Isomeren - sind in Tabelle 3 zusammengestellt.

**Tabelle 3**

| | T [°C] | Feed [g/h] | Rückführung [g/h] | Ausbeute [Gew.-%] | Selektivität C12 [%] |
|---|---|---|---|---|---|
| ohne Rückführung | 50 | 528 | 0 | 22,9 | 80,7 |
| | 50 | 142 | 0 | 36,8 | 75,6 |
| mit Rückführung | 50 | 45 | 406 | 46,1 | 73,8 |
| | 50 | 163 | 812 | 28,4 | 77,4 |

Erläuterung der Bezeichnungen in der Tabelle: Vgl. Beispiel 1.

## Patentansprüche

1. Verfahren zur Herstellung von im wesentlichen unverzweigten Oligomeren von Alkenen mit 4 bis 6 Kohlenstoffatomen ausgehend von den Alkenen oder einem solche Alkene enthaltende Kohlenwasserstoffströme an einem Nickel-haltigen heterogenen Katalysator in einem adiabatisch betriebenen Reaktor bei Temperaturen von 20 bis 300°C und Drücken von 1 bis 100 bar, **dadurch gekennzeichnet, dass** man einen ersten Teilstrom des Austrags aus dem Reaktor auf die Oligomeren aufarbeitet und den zweiten Teilstrom zusammen mit frischem Alken oder einem frischen, solche Alkene enthaltenden Kohlenwasserstoffstrom, in den Reaktor zurückführt.

2. Verfahren nach Anspruch 1, **dadurch gekennzeichnet, dass** man die bei der Aufarbeitung des ersten Teilstroms zurückgewonnenen, unumgesetzten Alkene mit 4 bis 6 Kohlenstoffatomen und gegebenenfalls die Alkane teilweise oder vollständig in den Reaktor zurückführt.

3. Verfahren nach den Ansprüchen 1 oder 2, **dadurch gekennzeichnet, dass** der erste Teilstrom 1 bis 50 Gew.-% des Austrags aus dem Reaktor beträgt.

4. Verfahren nach Anspruch 1 oder 2, **dadurch gekennzeichnet, dass** der erste Teilstrom 1 bis 20 Gew.-% des Austrags aus dem Reaktor beträgt.

5. Verfahren nach Anspruch 1 der 2, **dadurch gekennzeichnet, dass** der erste Teilstrom 1 bis 10 Gew.-% des Austrags beträgt.

6. Verfahren nach den Ansprüchen 1 bis 5, **dadurch gekennzeichnet, dass** man als Alkene enthaltende Kohlenwasserstoffströme solche mit mindestens einem Alken aus der Gruppe: n-Buten, n-Penten, n-Hexen einsetzt.

7. Verfahren nach den Ansprüchen 1 bis 6, **dadurch gekennzeichnet, dass** das Gewichtsverhältnis von zweitem Teilstrom zu frischem Alken oder dem frischen, ein solches Alken enthaltenden Kohlenwasserstoffstrom, 1 : 1 bis 50 : 1 beträgt.

8. Verfahren nach den Ansprüchen 1 bis 6, **dadurch gekennzeichnet, dass** das Gewichtsverhältnis von zweitem Teilstrom zu frischem Alken oder dem frischen, ein solches Alken enthaltenden Kohlenwasserstoffstrom, 2 : 1 bis 20 : 1 beträgt.

9. Verfahren nach den Ansprüchen 1 bis 6, **dadurch gekennzeichnet, dass** das Gewichtsverhältnis von zweitem Teilstrom zu frischem Alken oder dem frischen, ein solches Alken enthaltenden Kohlenwasserstoffstrom, 5 : 1 bis 20 : 1 beträgt.

10. Verfahren nach einem der Ansprüche 1 bis 9, bei dem ein Gewichtsverhältnis von Rückführstrom zu frisch zugeführtem Einsatzkohlenwasserstoffstrom von 0,5 bis 10, bezogen auf den stationären Zustand des Reaktionssystems, eingestellt wird.

## Claims

1. A process for preparing essentially unbranched oligomers of alkenes having from 4 to 6 carbon atoms starting from the alkenes or a hydrocarbon stream comprising such alkenes over a nickel-containing heterogeneous catalyst in an adiabatically operated reactor at from 20 to 300°C and pressures of from 1 to 100 bar, wherein a first substream of the output from the reactor is worked up to isolate the oligomers and the second substream is recirculated together with fresh alkene or a fresh hydrocarbon stream comprising such alkenes to the reactor.

2. A process as claimed in claim 1, wherein all or part of the unreacted alkenes having from 4 to 6 carbon atoms and recovered in the work-up of the first substream and, if appropriate, the alkanes are recirculated to the reactor.

3. A process as claimed in claim 1 or 2, wherein the first substream makes up from 1 to 50% by weight of the output from the reactor.

4. A process as claimed in claim 1 or 2, wherein the first substream makes up from 1 to 20% by weight of the output from the reactor.

5. A process as claimed in claim 1 or 2, wherein the first substream makes up from 1 to 10% by weight of the output.

6. A process as claimed in any of claims 1 to 5, wherein the alkene-containing hydrocarbon streams used are streams comprising at least one alkene selected from the group consisting of: n-butene, n-pentene and n-hexene.

7. A process as claimed in any of claims 1 to 6, wherein the weight ratio of the second substream to fresh alkene or the fresh hydrocarbon stream containing such an alkene is from 1:1 to 50:1.

8. A process as claimed in any of claims 1 to 6, wherein the weight ratio of the second substream to fresh alkene or the fresh hydrocarbon stream containing such an alkene is from 2:1 to 20:1.

9. A process as claimed in any of claims 1 to 6, wherein the weight ratio of the second substream to fresh alkene or the fresh hydrocarbon stream containing such an alkene is from 5:1 to 20:1.

10. A process as claimed in any of claims 1 to 9, wherein a weight ratio of recycle stream to freshly introduced hydrocarbon feed stream of from 0.5 to 10, based on the steady state of the reaction system, is set.

## Revendications

1. Procédé de préparation d'oligomères sensiblement non ramifiés d'alcènes à 4 à 6 atomes de carbone à partir de l'alcène ou d'un courant d'hydrocarbures contenant un tel alcène sur un catalyseur hétérogène contenant du nickel dans un réacteur en conditions adiabatiques à des températures de 20 à 300°C et sous des pressions comdel à 100 bars, **caractérisé en ce que** l'on régénère un premier courant partiel de la solution entraînée hors du réacteur sur les oligomères et que l'on réalimente le deuxième courant partiel conjointement avec un alcène frais ou un courant d'hydrocarbures frais contenant un tel alcène dans le réacteur.

2. Procédé selon la revendication 1, **caractérisé en ce que** l'on réalimente partiellement ou totalement l'alcène avec 4 à 6 atomes de carbones récupéré lors de la régénération du premier courant partiel non converti et le cas échéant les alcanes dans le réacteur.

3. Procédé selon la revendication 1 ou 2, **caractérisé en ce que** le premier courant partiel constitue 1 à 50 % en poids de la solution entraînée hors du réacteur.

4. Procédé selon la revendication 1 ou 2, **caractérisé en ce que** le premier courant partiel constitue 1 à 20 % en poids de la solution entraînée hors du réacteur.

5. Procédé selon la revendication 1 ou 2, **caractérisé en ce que** le premier courant partiel constitue 1 à 10 % en poids de la solution entraînée.

6. Procédé selon les revendications 1 à 5, **caractérisé en ce que** l'on met en oeuvre comme courant d'hydrocarbures contenant des alcènes un courant d'hydrocarbures contenant des alcènes avec au moins un alcène du groupe : n-butène, n-pentène, n-hexène.

7. Procédé selon les revendications 1 à 6, **caractérisé en ce que** le rapport pondéral du deuxième courant partiel par rapport à l'alcène frais ou au courant d'hydrocarbures frais contenant un tel alcène est compris entre 1:1 et 50:1.

8. Procédé selon les revendications 1 à 6, **caractérisé en ce que** le rapport pondéral du deuxième courant partiel par rapport à l'alcène frais ou au courant d'hydrocarbures frais contenant un tel alcène est compris entre 2:1 et 20:1.

9. Procédé selon les revendications 1 à 6, **caractérisé en ce que** le rapport pondéral du deuxième courant partiel par rapport à l'alcène frais ou au courant d'hydrocarbures frais contenant un tel alcène est compris entre 5:1 et 20:1.

10. Procédé selon l'une quelconque des revendications 1 à 9, dans lequel un rapport pondéral du courant de retour par rapport au courant d'hydrocarbures frais mis en oeuvre est réglé entre 0,5 et 10 par rapport à l'état stationnaire du système de réaction.
